# EUROPEAN PATENT APPLICATION

(11) **EP 1 334 724 A1**
(43) Date of publication of application: **13.08.2003**
(21) Application number: 01961337.1
(22) Date of filing: 04.09.2001
(51) Int. Cl.: A61K 31/565, A61K 31/57, A61K 9/70, A61K 47/32, A61K 47/44, A61P 5/30

(54) **COMPOSITIONS FOR EXTERNAL PREPARATIONS**

(30) Priority: 16.10.2000 JP 2000315179
(71) Applicant: HISAMITSU PHARMACEUTICAL CO. INC., Tosu-shi, Saga-ken 841-0017 (JP)
(72) Inventor: MAKI, M., c/o HISAMITSU PHARMACEUTICAL CO., Inc., Tosu-shi, Saga 841-0017 (JP); IKEURA, Y., c/o HISAMITSU PHARMACEUTICAL CO., Inc., Tosu-shi, Saga 841-0017 (JP)
(74) Representative: Cresswell, Thomas Anthony
(86) International application number: JP0107632
(87) International publication number: WO02032431

(57) **Abstract**

It is intended to provide compositions for external preparations (in particular, adhesive preparations) which are excellent in the skin permeability of the active ingredient, make it possible to give a compact preparation size and exert relieved skin irritation in percutaneous preparations, etc. to be used in, for example, hormone substitution therapy. Namely, compositions for external preparation which contain a vegetable oil and polyvinyl pyrrolidone as a sorbefacient and/or a dissolution aid; and adhesive preparations containing these compositions.

## Description

### Technical Field

The present invention relates to compositions for external preparations that are for percutaneous absorption of pharmaceutical ingredients. Precisely, it relates to percutaneous preparations having the advantages of good skin permeability of pharmaceutical ingredients and relieved skin irritation, especially to adhesive percutaneous preparations for medical use in hormone substitution therapy.

### Background Art

Percutaneous preparations are now available on the market for percutaneous therapy of various diseases . Above all, those for hormone substitution therapy for menopausal disorders and often for osteoporosis have produced good results. Estradiol, one typical estrogen used in hormone substitution therapy is secreted by the ovary during the women's genesial cycle. Accordingly, women in the perimenopause are essentially deficient in estradiol, therefore often suffering menopausal disorders, emmeniopathy, etc. At present, a therapy for relieving them through percutaneous application of estradiol is now on trial, in which the estradiol metabolism is reduced to make estradiol enters in blood. On the other hand, a study is now under investigation of percutaneous absorption of another hormone, progesterone to thereby retard the side effect in estradiol administration. A percutaneous preparation is proposed in JP-A 4-342532, which comprises pharmaceutical ingredients of estradiol and progesterone and for which the essential ingredient of the adhesive is an acrylic adhesive formed of 2-ethylhexyl acrylate and N-vinyl-2-pyrrolidone. In this, however, lactic acid and N-lauroylsarcosine that are used for absorption promoters much irritate skin, and the preparation is therefore not always satisfactory.

In JP-T 7-506562 (the term "JP-T" as used herein means a published Japanese translation of a PCT patent application), proposed is a percutaneous administration system with an absorption promoter composition of mixed vegetable oil that comprises coconut oil and soybean oil. However, many vegetable oils are often problematic in point of stability, and the number of different types of vegetable oils to be incorporated into one percutaneous preparation must be reduced as much as possible.

### Disclosure of the Invention

In view of the current situation as above, the present invention has been made for the purpose of 1) increasing the skin permeability of pharmaceutical ingredients (for making it possible to give a compact preparation size) and 2) relieving skin irritation in percutaneous preparations and others that are used, for example, for hormone substitution therapy.

### Best Mode for Carrying Out the Invention

The invention relates to a composition for external preparations, which contains vegetable oil and polyvinyl pyrrolidone as a sorbefacient and/or a dissolution aid.

The invention also relates to an adhesive preparation that contains the composition for external preparations.

Specifically, we, the present inventors have assiduously studied so as to solve the above-mentioned problems and, as a result, have found that a combination of vegetable oil, especially olive oil, and polyvinyl pyrrolidone is effective for promoting the percutaneous absorption of pharmaceutical ingredients and for relieving skin irritation. Having further studied, we have found that, when a substrate component selected from acrylic polymers or rubber polymers is combined with a composition component comprising vegetable oil and polyvinyl pyrrolidone, then percutaneous preparations, more concretely adhesive preparations having the advantage of good skin permeability of pharmaceutical ingredients and relieved skin irritation can be obtained. On the basis of these findings, we have completed the present invention.

The amount of the vegetable oil to be used in the composition for external preparations of the invention is from 1 to 25 % by mass, preferably from 3 to 15 % by mass, more preferably from 5 to 10 % by mass of the total amount of the composition.

If the vegetable oil is too much, it will bleed out of the preparations; but if too small, it will be ineffective for promoting the absorption of pharmaceutical ingredients.

Specific examples of the vegetable oil include, for example, almond oil, babassu oil, castor oil, Clark A oil, coconut oil, corn oil, cotton seed oil, jojoba oil, linseed oil, mustard oil, olive oil, palm oil, peanut oil, castoroil, safflower oil, sesame oil, soybean oil, sunflower seed oil, wheat germ oil, orange oil, etc. Above all, olive oil is especially preferred.

The amount of polyvinyl pyrrolidone to be used is from 1 to 10 % by mass , preferably from 2 to 9 % by mass , more preferably from 3 to 8 % by mass of the total amount of the composition.

If the amount of polyvinyl pyrrolidone is too small, the supersaturation is difficult to sustain for promoting the absorption of pharmaceutical ingredients; but if too large, the adhesiveness will be too high, therefore often causing the increase in skin irritation owing to corneal peeling, etc.

Polyvinyl pyrrolidone available on the market has a different weight-average molecular weight. In the invention, the polymer to be used preferably has a weight-average molecular weight of from 35000 to 80000, more preferably from 40000 to 60000. Having a weight-average molecular weight that falls within the range, the polymer produces especially good results in point of the skin permeability.

In case where the above-mentioned, more preferred range is employed for each of the vegetable oil and polyvinyl pyrrolidone, the absorption-promoting effect and the skin irritation-relieving effect could be the highest.

The composition for external preparations of the invention is effectively applicable to, for example, subcutaneous preparations such as ointment, gel, cream, lotion, aerosol, tape (plaster), epithem, etc.

The composition for external preparations of the invention can be widely used for any of these subcutaneous preparations, but above all, it is favorable for the substrate component for adhesive preparations. Of adhesive preparations, those for substantially water-free tape (plaster) are especially preferred for the composition of the invention.

The adhesive substrate to be used for the adhesive preparations of the invention includes polymers selected from the group consisting of acrylic polymers and rubber polymers.. One or more such polymers may be used herein either singly or as combined.

The amount of the acrylic polymer is from 40 to 95 % by mass, preferably from 50 to 90 % by mass, more preferably from 60 to 88 % by mass. Specific examples of acrylic polymers are, for example, National Starch & Chemical Corporation's acrylic adhesives (trade name, DURO-TAK80-1194, 80-2196, 80-1197, 387-2287, 387-2516, 87-2852), Monsanto's acrylic adhesives (trade name, GELVA-Multipolymer Solution GMS737, 788, 1151, 1430), Nippon Carbide Industry's acrylic adhesive (trade name, PE-300), etc.

For other acrylic adhesives, homopolymers or copolymers of alkyl (meth)acrylates in which the alkyl group has from 4 to 18 carbon atoms, or copolymers of alkyl (meth)acrylates of . that type with other functional monomers are preferably used.

Examples of the above-mentioned alkyl (meth)acrylates include, for example, butyl acrylate, isobutyl acrylate, hexyl acrylate, octyl acrylate, 2-ethylhexyl acrylate, isooctyl acrylate, decyl acrylate, isodecyl acrylate, lauryl acrylate, stearyl acrylate, methyl methacrylate, ethyl methacrylate, butyl methacrylate, isobutyl methacrylate, 2-ethylhexyl methacrylate, isooctyl methacrylate, decyl methacrylate, isodecyl methacrylate, lauryl methacrylate, stearyl methacrylate, etc.

Examples of the above-mentioned functional monomers are hydroxyl group-having monomers, carboxyl group-having monomers, amido group-having monomers, amino group-having monomers, pyrrolidone ring-having monomers, etc.

Examples of the hydroxyl group-having monomers are hydroxyalkyl (meth)acrylates such as 2-hydroxyethyl (meth)acrylate, hydroxypropyl (meth)acrylate, etc. Examples of the carboxyl group-having monomers are α,β-unsaturated carboxylic acids such as acrylic acid, methacrylic acid; monoalkyl maleates such as butyl maleate; maleic acid, fumaric acid, crotonic acid, etc. Maleic anhydride gives a copolymerization component like maleic acid.

Examples of the amido group-having monomers are acrylamide, methacrylamide; alkyl(meth)acrylamides such as dimethylacrylamide, diethylacrylamide, diethylmethacrylamide; N-alkoxymethyl(meth)acrylamides such as N-butoxymethylacrylamide, N-ethoxymethylacrylamide, N-ethoxymethylmethacrylamide; diacetonacrylamide, etc. Examples of the amino group-having monomers are dimethylaminoethyl acrylate, diethylaminoethyl methacrylate, etc. Examples of the pyrrolidone ring-having monomers are N-vinyl-2-pyrrolidone, etc.

The rubber polymers include thermoplastic elastomers and others, and their specific examples are styrene-isoprene-styrene block copolymers, polyisobutylene, etc. Some styrene-isoprene-styrene block copolymers are available on the market, and their examples are Shell Chemical's styrene-isoprene-styrene block copolymers (trade name, Califlex TR-1107, Califlex TR-1111), Nippon Synthetic Rubber's styrene-isoprene-styrene block copolymers (trade name, JSR5000, JSR5100), Nippon Zeon's styrene-isoprene-styrene block copolymer (trade name, Quintac 3421), etc.

Examples of commercial-available products of polyisobutylene are Exxon Chemical's polyisobutylene (trade name, Vistanex), BASF's polyisobutylene (trade name, Oppanol), etc.

The pharmaceutical ingredients that may be used in the composition for external preparations of the invention may be any of percutaneous, physiologically-active substances or so-called prodrugs that become physiologically active after percutaneously absorbed, and still may further be pharmaceutically-acceptable inorganic or organic addition salts.

Preferred examples of percutaneous pharmaceutical ingredients usable in the invention are female hormones such as estrogen, progesterone and their derivatives, etc.

Estrogen includes 17β-estradiol. estrone, estriol, equilin, equilenin and their derivatives. Of those, especially preferred is 17β-estradiol. Preferably, the amount of the estrogen to be used herein is in the range of from 0.5 to 10 % by mass of the total amount of the composition.

Progesterone includes progesterone, hydroxyprogesterone caproate, medroxyprogesterone acetate, dydrogesterone, chlormadinone acetate, ethisterone, dimethisterone, norethisterone, norethisterone acetate, norethisterone enanthate, ethynodiol acetate, megestrol acetate, allylestrenol and their derivatives. Of those, especially preferred are norethisterone or norethisterone acetate. Preferably, the amount of the progesterone to be used herein is in the range of from 1 to 10 % by mass of the total amount of the composition.

Other pharmaceutical ingredients than the above that may be used in the composition for external preparations of the invention are, for example, antiemetics (e.g., granisetron hydrochloride, azasetron hydrochloride, ondansetron hydrochloride, ramosetron hydrochloride, etc), remedies for pollakiuria (e.g., oxybutynin hydrochloride, etc.), Ca-antagonists (e.g., nifedipine, nisoldipine, nicardipine, nitredipine, etc.), corticosteroids (e.g., hydrocortisone, prednisolone, clobetasol propionate, etc.), anti-inflammatory sedatives (e.g., celecoxib, rofecoxib, ibuprofen, naproxen, diclofenac, piroxicam, indomethacin, ketoprofen, flurbiprofen, felbinac, ketorolac, etc.), hypnotic sedatives (e.g., phenobarbital, triazolam, nitrazepam, lorazepam, etc.), tranquilizers (e.g., fluphenazine, diazepam, chlorpromazine, etc.), antihypertensives (e.g., clonidine, pindolol, propranolol, nitrendipine, metoprolol, etc.), hypotensive diuretics (e.g., hydrothiazide, etc.), antibiotics (e.g., penicillin, tetracycline, erythromycin, chloramphenicol, etc.), narcotics (e.g., lidocaine, dibucaine hydrochloride, ethyl aminobenzoate, etc.), antimicrobial substances (e.g., benzalkonium chloride, clotrimazole, etc.), vitamins (e.g., vitamins A, B₁, B₂, C, D, K, etc.), anticonvulsants (e.g., nitrazepam, etc.), coronary vasodilatives (e.g., nitroglycerin, isosorbide nitrate, etc.), antihistaminics (e.g., diphenhydramine, chlorpheniramin, etc.), spasmolytics (e.g., tulobuterol hydrochloride, salbutamol, ketotifen fumarate, tranilast, isoproterenol hydrochloride, etc.), antidepressants (e.g., doxepin, etc.), cerebral circulation improvers (e.g., hydergine, ifenprodil, etc.), antitumor agents (e.g., 5-fluorouracil, etc.), muscular relaxants (e.g., eperisone, dantrolene, etc.), analgesics (e.g., fentanyl, morphine, etc.), polypeptidic hormones (e.g., luteinizing hormone-releasing hormone (LH-RH), thyrotropin-releasing hormone (TRH), etc.), peripheral vasodilatives, immunomodulatory agents (e.g., ciclosporin, tacrolimus hydrate, mycophenolate mofentil, mizoribine, auranofin, lobenzarit, etc.), cholagogues (e.g., ursodesoxycholic acid, etc.), narcotics (e.g., morphine hydrochloride, dihydrocodeine phosphate, etc.), no-smoking aids (e.g., nicotine, etc.), anti-obesity agents (e.g., sibutramin hydrochloride, mazindol, etc.), diuretics (e.g., hydroflumethiazide, etc.), medicines for diabetes (e.g., glimepiride, troglitazone, nateglinide, repaglinide, pioglitazone hydrochloride, tolubutamide, etc.), remedies for gout (e.g., colchicine, etc.), remedies for Parkinson's disease (e.g., amantadine, levodopa, etc.), antivertiginous agents (e.g., difenidol, betahistine, etc.), refrigerants (e.g., fennel oil, d-camphor, dl-camphor, cinnamon oil, mentha water, mentha oil, bergamot oil, d-borneol, 1-menthol, borneol, etc.)

The amount of the pharmaceutical ingredient to be used differs, depending on the object thereof to be incorporated into the composition, but is generally an effective amount for therapy preferably falling between 0.1 and 10 % by mass of the total weight of the composition. Not causing any unfavorable interaction therebetween, two or more different types of these pharmaceutical ingredients may be optionally combined for use herein.

The composition for external preparations of the invention and the adhesive preparation that contains it may contain, in addition to the components mentioned hereinabove, any of softeners, adhesiveness imparting resins, etc.

Examples of the softeners are liquid paraffin, polybutene, process oil, etc.

Examples of the adhesiveness imparting resins are alicyclic saturated hydrocarbon resins (e.g., Arkon P-100 (trade name by Arakawa Chemical Industry)), rosin esters (e.g., Ester Gum (trade name by Arakawa Chemical Industry)), hydrogen-alicyclic hydrocarbons (e.g., Escorez 5300 (trade name by Exxon Chemical)), terpene-type hydrogenated resins (e.g., Clearon P-105 (trade name by Yasuhara Chemical)), hydrogenated rosin glycerin esters (e.g., Foral 85, Foral 105 (trade name by Rika Hercules)), dibasic acid-modified rosin esters (e.g., Pentalyn 4741 (trade name by Rika Hercules)), etc.

If desired, two or more different types of these adhesiveness imparting resins may be mixed for use herein.

Further if desired, the composition may contain any other known additives for controlling its adhesiveness, safety and stability of the composition. Concretely, if desired, it may contain a suitable amount of any of absorbing polymers such as Sumikagel SP-520 (trade name by Sumitomo Chemical)), Aquakeep 10SH (trade name by Sumitomo Seika)), Arasorb 800F (trade name by Arakawa Chemical Industry)), Sunwet 1M-1000MPS (trade name by Sanyo Chemical)); inorganic fillers such as zinc oxide, calcium carbonate, titanium dioxide, silica; other dissolution aids such as glycerin fatty acid esters, e.g., Excel (trade name by Kao), Crotamiton, etc.; other absorption promoters such as aliphatic alcohols, e.g., KALCOL (trade name by Kao); moisturizers such as triethyl citrate, polyethylene glycol, glycerin, etc.

In case where the composition for external preparations of the invention is used for adhesive preparations, films that serve as the support for them may be flexible or non-flexible, and they must have such properties as being excellent in so-called barrier properties for preventing pharmaceutical ingredients from leaking out, evaporating away and adsorbed by any others. Preferably, they are flexible in some degree when the adhesive preparations are applied to skin.

Satisfying the above-mentioned requirements, the material of the support is not specifically defined. Concretely, its examples are aluminium, ethylene-vinyl acetate copolymer (e.g., Scotchpack 9733 (trade name by 3M Health Care)) or its saponified products, cellulose acetate, cellulose, nylon, polyester (e.g., Binersheet (trade name by Fujimori Industry)), polyethylene (e.g., COTran 9720 (trade name by 3M Health Care)), polyvinylidene chloride, polyvinyl chloride, polycarbonate, polyurethane (e.g. COTran 9701 (trade name by 3M Health Care)), polyvinyl alcohol, polypropylene (e.g., COTran 9725 (trade name by 3M Health Care)), etc. These materials are formed in film, or, if desired, their film may be laminated with paper or cloth to give laminated films, or may be processed for aluminium deposition (Scotchpack 1109 (trade name by 3M Health Care)) or ceramic deposition thereon to enhance the barrier properties of the thus-processed films.

The film to be a release liner layer must have the function of preventing the pharmaceutical ingredient from leaking out or evaporating away during the storage of the preparations, and the release liner layer must be peelable before use. Concretely, the material of the release liner film usable herein includes aluminium, cellulose, polyester (e.g., Binersheet (trade name by Fujimori Industry)), polyethylene, polypropylene, etc. If desired, the films may be laminated. Further, the film surface may be processed with silicone, fluorocarbon or the like or any known additive may be incorporated into the liner material to thereby control the releasability and the barrier properties of the film. The release liner may have a pinch fin for its better handling in peeling.

A method for producing the adhesive preparations of the invention is described. For example, the adhesive preparations of the invention may be produced by dissolving pharmaceutical ingredients, vegetable oil, polyvinyl pyrrolidone and others in the above-mentioned, commercially-available acrylic adhesive, spreading the resulting solution on a support, removing the organic solvent, covering it with a liner, and cutting it into products of a desired shape. Alternatively, the solution may be once spread on a lubricated film, then the solvent is removed, and this may be transferred onto a suitable support under pressure to be a product.

Still another method comprises dissolving the above-mentioned pharmaceutical ingredients and substrate ingredients and others in an organic solvent such as ethanol, hexane, toluene or ethyl acetate, then spreading the resulting solution on a support, removing the organic solvent, covering it with a liner, and cutting it into products of a desired shape. Alternatively, the solution may be once spread on a lubricated film, then the solvent is removed, and this may be transferred onto a suitable support under pressure to be a product.

### Examples

The adhesive percutaneous preparation that contains the composition for external preparations of the invention is described in more detail with reference to the following Examples and Test Examples. Needless-to-say, however, the invention should not be limited to these Examples.

In the following Examples and Comparative Examples, numerals are all in terms of % by mass.

### Example 1:

| | |
|---|---|
| Acrylic adhesive (trade name, DURO-TAK80-2196) | 72.0 |
| Vegetable oil (sesame oil) | 5.0 |
| Polyvinyl pyrrolidone (weight-average molecular weight, 44000-54000) (trade name, Kollidon 30) | 5.0 |
| Hydrogenated rosin glycerin ester (trade name, Foral 85) | 5.0 |
| Estradiol | 5.0 |
| Norethisterone acetate | 8.0 |

According to the above-mentioned production method, all these pharmaceutical ingredients and substrate ingredients were dissolved, then the resulting solution was spread on a polyethylene terephthalate film, the solvent was removed by drying, and this was covered with a silicone-processed polyethylene terephthalate liner to be an adhesive preparation.

### Example 2:

| | |
|---|---|
| Acrylic adhesive (trade name, GMS788) | 63.5 |
| Vegetable oil (soybean oil) | 25.0 |
| Polyvinyl pyrrolidone (weight-average molecular weight, | |
| 44000-54000) (trade name, Kollidon 30) | 10.0 |
| Estradiol | 0.5 |
| Norethisterone acetate | 1.0 |

According to the above-mentioned production method, all these pharmaceutical ingredients and substrate ingredients were dissolved, then the resulting solution was spread on a silicone-processed polyethylene terephthalate liner, the solvent was removed by drying, and this was transferred onto a non-flexible polyethylene film under pressure to be an adhesive preparation.

### Example 3:

| | |
|---|---|
| Acrylic adhesive (trade name, GMS737) | 69.0 |
| Vegetable oil (palm oil) | 1.0 |
| Polyvinyl pyrrolidone (weight-average molecular weight, 44000-54000) (trade name, Kollidon 30) | 10.0 |
| Estradiol | 10.0 |
| Norethisterone acetate | 10.0 |

According to the above-mentioned production method, all these pharmaceutical ingredients and substrate ingredients were dissolved, then the resulting solution was spread on a silicone-processed polyethylene terephthalate liner, the solvent was removed by drying, and this was transferred onto a non-flexible polyethylene film under pressure to be an adhesive preparation.

### Example 4:

| | |
|---|---|
| Acrylic adhesive (trade name, DURO-TAK87-2852) | 82.0 |
| Vegetable oil (castor oil) | 10.0 |
| Polyvinyl pyrrolidone (weight-average molecular weight, 44000-54000) (trade name, Kollidon 30) | 1.0 |
| Estradiol | 2.0 |
| Norethisterone | 5.0 |

According to the above-mentioned production method, all these pharmaceutical ingredients and substrate ingredients were dissolved, then the resulting solution was spread on a polyethylene terephthalate film, the solvent was removed by drying, and this was covered with a silicone-processed polyethylene terephthalate liner to be an adhesive preparation.

### Example 5:

| | |
|---|---|
| Acrylic adhesive (trade name, DURO-TAK387-2516) | 70.0 |
| Vegetable oil (olive oil) | 10.0 |
| Polyvinyl pyrrolidone (weight-average molecular weight, 44000-54000) (trade name, Kollidon 30) | 7.0 |
| Estradiol | 4.0 |
| Norethisterone acetate | 9.0 |

According to the above-mentioned production method, all these pharmaceutical ingredients and substrate ingredients were dissolved, then the resulting solution was spread on a polyethylene terephthalate film, the solvent was removed by drying, and this was covered with a silicone-processed polyethylene terephthalate liner to be an adhesive preparation.

### Comparative Example 1:

Constitutive ingredients were prepared in the same manner as in Example 5 except that vegetable oil and polyvinyl pyrrolidone were not used and the amount of the acrylic adhesive was changed to 87.0. According to the above-mentioned production method, all the pharmaceutical ingredients and the substrate ingredients were dissolved, then the resulting solution was spread on a polyethylene terephthalate film, the solvent was removed by drying, and this was covered with a silicone-processed polyethylene terephthalate liner to be an adhesive preparation.

### Comparative Example 2:

Constitutive ingredients were prepared in the same manner as in Example 5 except that vegetable oil was not used and the amount of the acrylic adhesive was changed to 80.0. According to the above-mentioned production method, all the pharmaceutical ingredients and the substrate ingredients were dissolved, then the resulting solution was spread on a polyethylene terephthalate film, the solvent was removed by drying, and this was covered with a silicone-processed polyethylene terephthalate liner to be an adhesive preparation.

### Comparative Example 3:

Constitutive ingredients were prepared in the same manner as in Example 5 except that polyvinyl pyrrolidone was not used, castor oil was used as the vegetable oil and the amount of the acrylic adhesive was changed to 77.0. According to the above-mentioned production method, all the pharmaceutical ingredients and the substrate ingredients were dissolved, then the resulting solution was spread on a polyethylene terephthalate film, the solvent was removed by drying, and this was covered with a silicone-processed polyethylene terephthalate liner to be an adhesive preparation.

### Comparative Example 4:

Constitutive ingredients were prepared in the same manner as in Example 5 except that a fatty acid ester (isopropyl myristate) was used in place of the vegetable oil, polyvinyl pyrrolidone having a molecular weight (1000000 to 1500000) not falling within the range defined herein was used and the amount of the acrylic adhesive was changed to 75.0.

According to the above-mentioned production method, all the pharmaceutical ingredients and the substrate ingredients were dissolved, then the resulting solution was spread on a polyethylene terephthalate film, the solvent was removed by drying, and this was covered with a silicone-processed polyethylene terephthalate liner to be an adhesive preparation.

### Test Example 1 - Effect of retarding crystal precipitation:

The test pieces of Examples 1 to 5 and those of Comparative Examples 1 and 4 were stored at 60°C for 1 month, and checked for crystal precipitation therein. The result is given in Table 1.

**Table 1**

| Test Piece | After stored at 60°C for 1 month |
|---|---|
| Example 1 | no crystal precipitated |
| Example 2 | no crystal precipitated |
| Example 3 | no crystal precipitated |
| Example 4 | no crystal precipitated |
| Example 5 | no crystal precipitated |
| Comparative Example 1 | crystal precipitated |
| Comparative Example 4 | crystal precipitated |

As is obvious from Table 1, there occurred no problem with the test pieces of Examples 1 to 5, but some crystal formation was found in the test pieces of Comparative Examples 1 and 4.

### Test Example 2 - Test for skin permeability:

Test pieces of Examples 1 and 5 and those of Comparative Examples 1 to 3 were tested, for which a Franz diffusion cell of 1 cm² was used. Each test piece was applied onto the skin of the back of each hairless mouse (10-weeks old, female) for testing it for its skin permeability at 37°C. The receptor liquid was sampled at predetermined intervals after the start of the test, and immediately a fresh receptor liquid was replenished. The amount of the pharmaceutical ingredients having transferred into the sampled receptor liquid was measured through high-performance liquid chromatography. The number of the samples of each test piece tested herein was 3. The maximum transmission rate of estradiol (E₂) and norethisterone (NET) in every sample is shown in Table 2.

**Table 2**

| Test Piece | Maximum Transmission Rate (µg/cm²/hr) | |
|---|---|---|
| | Estradiol (E₂) | Norethisterone (NET) |
| Example 1 | 0.7 | 0.8 |
| Example 5 | 0.9 | 0.9 |
| Comparative Example 1 | 0.3 | 0.4 |
| Comparative Example 2 | 0.4 | 0.4 |
| Comparative Example 3 | 0.4 | 0.4 |

As is obvious from Table 2, the test pieces of Examples 1 and 5 showed good permeability, but the permeability through those of Comparative Examples 1 to 3 was low.

### Test Example 3 - Test for skin irritation:

The test pieces of Examples 1 and 5 and Comparative Example 4 were tested for skin irritation according to the method mentioned below. Each test piece was attached to the arm of each of 10 panelist (healthy male). and tested for skin irritation after 48 hours. The result is given in Table 3.

**Table 3**

| Test Piece | Index of Skin Irritation (SI) |
|---|---|
| Example 1 | 20 |
| Example 5 | 23 |
| Comparative Example 4 | 35 |

As is obvious from Table 3, the skin irritation with the test pieces of Examples 1 and 5 is significantly lower than that with the test piece of Comparative Example 4.

### Industrial Applicability

The composition for external applications of the invention and the adhesive preparations with it therein contain vegetable oil and polyvinylpyrrolidone as a sorbefacient and/or a dissolution aid, and therefore have the excellent advantages of efficiently increased skin permeability of pharmaceutical ingredients and relived skin irritation. In addition, the composition is further effective for stabilizing the preparations. Accordingly, the invention is extremely useful for compositions for external applications and for adhesive preparations, in particular for therapeutic adhesive percutaneous preparations in hormone substitution therapy.

## Claims

1. A substrate composition for percutaneous absorption containing vegetable oil and polyvinyl pyrrolidone as an absorption accelerator and/or a solubilizer.

2. The composition for percutaneous absorption as claimed in claim 1, which contains from 1 to 25 % by mass of vegetable oil and from 1 to 10 % by mass of polyvinyl pyrrolidone.

3. The composition for percutaneous absorption as claimed in claim 1 or 2, wherein the vegetable oil is olive oil.

4. The composition for percutaneous absorption as claimed in any one of claims 1 to 3, wherein the polyvinyl pyrrolidone has a weight-average molecular weight of from 35000 to 80000.

5. The composition for percutaneous absorption as claimed in any one of claims 1 to 4, which contains a percutaneous pharmaceutical ingredient.

6. The composition for percutaneous absorption as claimed in claim 5, wherein the pharmaceutical ingredient is estrogen and/or progesterone.

7. The composition for percutaneous absorption as claimed in claim 6, wherein the estrogen is 17β-estradiol.

8. The composition for percutaneous absorption as claimed in claim 7, wherein the content of 17β-estradiol is from 0.5 to 10 % by mass.

9. The composition for percutaneous absorption as claimed in claim 6, wherein the progesterone is norethisterone or norethisterone acetate.

10. The composition for percutaneous absorption as claimed in claim 9, wherein the content of norethisterone or norethisterone acetate is from 1 to 10 % by mass.

11. An adhesive preparation containing the composition for percutaneous absorption of claim 5.

12. The adhesive preparation as claimed in claim 11, which contains, as the adhesive substrate, one or more polymers selected from a group consisting of acrylic polymers and rubber polymers.

13. The adhesive preparation as claimed in claim 11 or 12, which contains, as the pharmaceutical ingredient, estrogen and/or progesterone.

14. The adhesive preparation as claimed in claim 13, wherein the estrogen is 17β-estradiol.

15. The adhesive preparation as claimed in claim 14, wherein the content of 17β-estradiol is from 0.5 to 10 % by mass.

16. The adhesive preparation as claimed in claim 13, wherein the progesterone is norethisterone or norethisterone acetate.

17. The adhesive preparation as claimed in claim 16, wherein the content of norethisterone or norethisterone acetate is from 1 to 10 % by mass.
